# EUROPEAN PATENT APPLICATION

(11) **EP 4 321 178 A1**
(43) Date of publication of application: **14.02.2024**
(21) Application number: 22784920.5
(22) Date of filing: 05.04.2022
(51) Int. Cl.: A61K 47/64, A61K 39/215, A61K 39/29, A61K 39/42, A61K 35/16, A61P 31/14, A61P 31/20, A61K 39/00

(54) **VACCINE COMPOSITION AGAINST CORONAVIRUS**

(30) Priority: 05.04.2021 KR 20210044328
(71) Applicant: LG Chem, Ltd., Seoul 07336 (KR)
(72) Inventor: NOH, Yoon Ae, Daejeon 34122 (KR); LEE, Seungwon, Daejeon 34122 (KR); KIM, Yeonchul, Daejeon 34122 (KR); HUR, Ji Yeon, Daejeon 34122 (KR); NA, Kyubong, Daejeon 34122 (KR)
(74) Representative: Goddar, Heinz J.
(86) International application number: PCT/KR2022/004887
(87) International publication number: WO 2022/216011

(57) **Abstract**

The present invention relates to: a conjugate in which a receptor-binding domain (RBD) of coronavirus and a hepatitis B surface antigen (HBsAg) are linked; virus-like particles including the conjugate; and a coronavirus vaccine composition containing the conjugate and/or virus-like particles.

## Description

### [TECHNICAL FIELD]

### Cross-reference to related applications

The present application claims the benefit of the priority based on Korean Patent Application No. 10-2021-0044328 filed on April 5, 2021, and the entire contents disclosed in the document of the corresponding Korean patent application are incorporated in the present description as a part.

The present description relates to a conjugate in which a receptor-binding domain (RBD) of coronavirus and a hepatitis B surface antigen (HBsAg) are connected, a virus-like particle comprising the conjugate, and a vaccine composition against coronavirus comprising the conjugate and/or virus-like particle.

### [BACKGROUND ART]

Coronavirus is a virus species belonging to the family *Coronaviridae*, and is a positive-sense RNA virus that infects humans and animals and causes respiratory, gastrointestinal and/or nervous system diseases. As the coronavirus, severe acute respiratory syndrome coronavirus (SARS-CoV), middle east respiratory syndrome coronavirus (MERS-CoV), SARS-CoV-2 (COVID-19) and the like may be exemplified, and it may cause serious infectious diseases in humans.

In particular, for the novel coronavirus newly discovered in 2019 (SARS-CoV-2; COVID-19), vaccines using mRNA, vaccines using adenovirus, and the like have been urgently approved and are in use, but their efficacy against mutant viruses is low.

There is a need for development of a vaccine to effectively prevent infection of coronavirus, particularly, novel coronavirus or mutant viruses thereof.

### [DISCLOSURE]

### [TECHNICAL PROBLEM]

Provided is SARS-CoV-2 RBD-HBsAg conjugate, in which a receptor binding domain (RBD) of a spike protein of coronavirus, particularly, SARS-CoV-2 (COVID-19) (hereinafter, SARS-CoV-2 RBD') and a hepatitis B surface antigen (HBsAg) are linked through or without a linker.

The SARS-CoV-2 RBD may be a wild-type or mutant.

The hepatitis B surface antigen (HBsAg) or the conjugate may be in a form of virus-like particle (VLP).

In the conjugate, the SARS-CoV-2 RBD may be linked to the hepatitis B surface antigen in a VLP form through or without a linker, or the SARS-CoV-2 RBD may form a VLP form in which SARS-CoV-2 RBD is exposed on the particle surface by self-assemble of the hepatitis B surface antigen which is linked thereto through or without a linker. When the conjugate is in a virus-like particle form, the SARS-CoV-2 RBD may be located on this particle surface.

In an embodiment, the SARS-CoV-2 RBD and hepatitis B surface antigen may be linked through a chemical linker. The chemical linker may be selected from crosslinking agent (coupling agent) compounds having a functional group capable of linking a protein. In an example, the chemical linker may be a compound having at least one functional group selected from the group consisting of functional groups including oxygen, nitrogen, or sulfur, for example, aldehyde, imide, ester, thiol, sulfonyl, and the like, and for example, may be one or more selected from the group consisting of glutaraldehyde, SMPH (succinimidyl-6-((b-maleimidopropionamido)hexanoate), SMPB (Succinimidyl 4-(p-maleimidophenyl)butyrate), Sulfo-SMPB, Sulfo-EMCS [Sulfo-(N-ε-maleimidocaproyl-oxysulfosuccinimide ester)], Sulfo-GMBS [Sulfo-(N-γ-maleimidobutyryl-oxysuccinimide ester)], SM[PEG]n (NHS-PEGn-Maliemide), AMAS (N-(α-Maleimidoacetoxy)-succinimide ester), BMPS (N-(β-Maleimidopropyloxy)succinimide ester), EMCA (N-ε-Maleimidocaproic acid), EMCS (N-(ε-Maleimidocaproyloxy)succinimide ester), GMBS (N-(γ-Maleimidobutyryloxy)succinimide ester), LC-SMCC (Succinimidyl 4-(N-maleimidomethyl) cyclohexane-1-carboxy-(6-amidocaproate)), MBS (m-Maleimidobenzoyl-N-hydroxysuccinimide ester), SBAP (Succinimdyl 3-(bromoacetamido)propionate), SIA (N-succinimidyl iodoacetate), SIAB (N-Succinimidyl(4-iodoacetyl)aminobenzoate), Sulfo-KMUS (N-(κ-Maleimidoundecanoyloxy)sulfosuccinimide ester), Sulfo-MBS (m-Maleimidobenzoyl-N-hydroxysulfosuccinimide ester), SUlfo-SIAB (Sulfosuccinimidyl(4-iodo-acetyl)aminobenzoate), and the like, but not limited thereto.

The conjugate may form a virus-like particle (VLP). The VLP may be in a form in which SARS-CoV-2 RBD is located (exposed) on the particle surface, and as aforementioned, may be formed by the hepatitis B surface antigen. Accordingly, another embodiment provides a virus-like particle comprising the conjugate.

The conjugate and/or virus-like particle may function as an immunogen or antigenic substance in a living body.

Another embodiment provides a nucleic acid molecule for producing the conjugate. The nucleic acid molecule may comprise a combination of a SARS-CoV-2 RBD-coding polynucleotide and a hepatitis B surface antigen-coding polynucleotide.

Another embodiment provides a recombinant vector comprising the nucleic acid molecule. The recombinant vector may be used as an expression vector in a host cell. The recombinant vector may comprise the SARS-CoV-2 RBD-coding polynucleotide and hepatitis B surface antigen-coding polynucleotide in one vector or comprise them in two vectors, respectively.

Another embodiment provides a recombinant cell comprising the nucleic acid molecule or recombinant vector. The recombinant cell may be obtained by introducing the recombinant vector into a host cell.

Another embodiment provides a vaccine composition against coronavirus, comprising the conjugate and/or virus-like particle. The vaccine composition may further comprise an adjuvant.

Another embodiment provides a use of the conjugate and/or virus-like particle for immunization against coronavirus or for preparation of the vaccine composition against coronavirus. The vaccine composition may further comprise an adjuvant.

Another embodiment provides a method for immunization against coronavirus, comprising administering the conjugate and/or virus-like particle or the vaccine composition to a subject in need of immunization against coronavirus.

Another embodiment provides a composition for prevention of infection or disease and/or symptom related to infection of coronavirus, comprising the conjugate and/or virus-like particle, or the vaccine composition.

Another embodiment provides a use of the conjugate and/or virus-like particle, or the vaccine composition, for prevention of infection or disease and/or symptom related to infection of coronavirus, or for preparation of a composition for prevention of infection or disease and/or symptom related to infection of coronavirus.

Another embodiment provides a method for prevention of infection of coronavirus or disease and/or symptom related to infection of coronavirus, comprising administering the conjugate and/or virus-like particle or the vaccine composition, to a subject in need of prevention of infection of coronavirus or disease and/or symptom related to infection of coronavirus.

Another embodiment provides a pharmaceutical composition for treatment of infection of coronavirus or disease and/or symptom related to infection of coronavirus, comprising an antibody binding to the conjugate and/or virus-like particle, or blood or serum isolated from a subject administered with the conjugate and/or virus-like particle. The antibody may be isolated from blood or serum isolated from a subject administered with the conjugate and/or virus-like particle.

Another embodiment provides a use of an antibody binding to the conjugate and/or virus-like particle, or blood or serum isolated from a subject administered with the conjugate and/or virus-like particle, for treatment of infection of coronavirus or disease and/or symptom related to infection of coronavirus, or for preparation of a pharmaceutical composition for treatment of infection of coronavirus or disease and/or symptom related to infection of coronavirus. The antibody may be isolated from blood or serum isolated from a subject administered with the conjugate and/or virus-like particle.

Another embodiment provides a method for treatment of infection of coronavirus or disease and/or symptom related to infection of coronavirus, comprising administering an antibody binding to the conjugate and/or virus-like particle, or blood or serum isolated from a subject administered with the conjugate and/or virus-like particle, to a patient in need of treatment of infection of coronavirus or disease and/or symptom related to infection of coronavirus. The antibody may be isolated from blood or serum isolated from a subject administered with the conjugate and/or virus-like particle.

Another embodiment provides a method for preparation of a conjugate comprising SARS-CoV-2 RBD and a hepatitis B surface antigen, a virus-like particle comprising the conjugate, or a vaccine composition against coronavirus comprising them, comprising expressing a nucleic acid molecule encoding the SARS-CoV-2 RBD and hepatitis B surface antigen in a host cell. The method may further comprise adding a chemical linker, before, after or at the same time with the expressing.

The coronavirus may be SARS-CoV-2 virus.

### [TECHNICAL SOLUTION]

### Definition of terms

In the present description, the term "vaccine," which is a biological agent containing an antigen inducing immunity in a living body, may refer to an immunogenic or antigenic substance capable of conferring immunity to a living body when it is administered to a human or animal for prevention of infectious disease.

In the present description, the term "coronavirus vaccine composition or vaccine composition against coronavirus" may refer to a pharmaceutical composition causing an immune response against coronavirus, and unless otherwise described, the term may be used as the same meaning as a coronavirus immunogenic composition, and may be interchangeable thereto.

As described in the present description, the expression of "comprising (a prescribed component)" or "consisting of (a prescribed component)" may refer to essentially comprising the described component, and as long as the effect is equivalent to the desired effect, it may not exclude comprising an additional and/or auxiliary component.

In the present description, the phrases that a nucleic acid molecule (may be interchangeable with "gene") or polypeptide (may be interchangeable with "protein") "comprises a specific nucleic acid sequence or amino acid sequence," "consists of a specific nucleic acid sequence or amino acid sequence," or "is represented by a specific nucleic acid sequence or amino acid sequence" may be interchangeably used with one another to equivalent meaning, and may refer to that the nucleic acid molecule or polypeptide essentially comprises the specific nucleic acid sequence or amino acid sequence, and may be interpreted as including "a substantially equivalent sequence" having a mutation (deletion, substitution, modification and/or addition) in the specific nucleic acid sequence or amino acid sequence with maintaining the original function and/or desired function of the nucleic acid molecule or polypeptide (or not excluding such mutant).

Hereinafter, the present invention will be described in more detail:

### SARS-CoV-2 RBD-HBsAg conjugate

An embodiment provides a SARS-CoV-2 RBD-HBsAg conjugate, comprising a receptor binding domain of a spike protein of coronavirus (for example, SARS-CoV-2 RBD) and a hepatitis B surface antigen. In the conjugate, the receptor binding domain of a spike protein of coronavirus and hepatitis B surface antigen may be linked through or without a linker. In an embodiment, the conjugate may further comprise a chemical linker. In this case, the receptor binding domain of a spike protein of coronavirus and hepatitis B surface antigen may be linked through the chemical linker.

In a specific embodiment, the coronavirus may be SARS-CoV-2 (COVID-19).

The SARS-CoV-2 RBD and hepatitis B surface antigen may be produced by recombinant or chemical synthesis, and in case of recombinant synthesis, they may be produced alone, respectively, or produced in a conjugate form together, in an appropriate host cell, but not limited thereto.

The SARS-CoV-2 (COVID-19) has a spike protein (GenBank: QHD43416.1; 1273 aa; SEQ ID NO: 1) on the virus surface, and the RBD (Receptor binding domain) of the spike protein binds to a receptor of a host cell (human cell) and enters inside a cell, and therefore, the SARS-CoV-2 (COVID-19) RBD can function as an antigen of the SARS-CoV-2 vaccine.

The SARS-CoV-2 RBD may be an amino acid sequence region from the 319th (R319) to the 541th (F541) from the N-terminus, based on the spike protein of SEQ ID NO: 1 or a part or extension thereof.

In one embodiment, the SARS-CoV-2 RBD may be expressed by:
an amino acid sequence (wild-type or original; SEQ ID NO: 2) from the 319th to 541th from the N-terminus based on the spike protein of SEQ ID NO: 1,
a part thereof (for example, a polypeptide fragment in which 10 to 30, 10 to 25, or 10 to 20 (e.g., 10, 11, 12, 13, 14, 15, 16, 17, 18, 19 or 20) consecutive or nonconsecutive amino acids are deleted at the N-terminus, C-terminus, or both termini of SEQ ID NO: 2) (when both termini are deleted, the number of amino acids deleted at each terminus may be same or different from each other); e.g., a polypeptide fragment comprising at least 190 consecutive amino acids comprising SEQ ID NO: 5 in SEQ ID NO: 2),
an extension thereof (for example, a polypeptide extension in which 10 to 30, 10 to 25, or 10 to 20 (for example, 5, 6, 7, 8, 9, 10, 11, 12, 13, 14, 15, 16, 17, 18, 19 or 20) amino acids are added (elongated) at the N-terminus, C-terminus or each of both termini of SEQ ID NO: 2), or
a mutant amino acid sequence having a mutation in the above amino acid sequences.

In a specific embodiment, when the SARS-CoV-2 RBD is conjugated to the hepatitis B surface antigen through free cysteine (Free Cys) (for example, using a linker such as SMPH, SMPB, and the like), the polypeptide fragment or extension may comprise one or more cysteines selected from the group consisting of C301, C336, C361, C379, C432, C391, C480, C488, C525 and C538, for example, C301, C538 or both of them, in a free from, but not limited thereto.

The mutation of the SARS-CoV-2 RBD may comprise one or more (for example, 1, 2, 3, 4 or 5) selected from the group consisting of substitution of the 417th amino acid residue, lysine (K417) or an amino acid residue corresponding thereto with another amino acid (e.g., K417N or K417T); substitution of the 452th amino acid residue, leucine (L452) or an amino acid residue corresponding thereto with another amino acid (e.g., L452R); substitution of the 477th amino acid residue, serine (S477) or an amino acid residue corresponding thereto with another amino acid (e.g., S477N); substitution of the 484th amino acid residue, glutamic acid (E484) or an amino acid residue corresponding thereto with another amino acid (e.g., E484K); and substitution of the 501th amino acid residue, asparagine (N501) or an amino acid residue corresponding thereto with another amino acid (e.g., N501Y), from the N-terminus, based on the spike protein of SEQ ID NO: 1.

More specifically, the mutation of the SARS-CoV-2 RBD may comprise:
substitution of the 501th amino acid residue, asparagine (N501) or an amino acid residue corresponding thereto with another amino acid (e.g., N501Y) (for example, England mutation SARS-CoV-2 virus, B.1.1.7 type);
one or more (for example, 1, 2 or 3) selected from the group consisting of substitution of the 417th amino acid residue, lysine (K417) or an amino acid residue corresponding thereto with another amino acid (e.g., K417N), substitution of the 484th amino acid residue, glutamic acid (E484) or an amino acid residue corresponding thereto with another amino acid (e.g., E484K), and substitution of the 501th amino acid residue, asparagine (N501) or an amino acid residue corresponding thereto with another amino acid (e.g., N501Y) (for example, the Republic of South Africa mutation SARS-CoV-2 virus, B.1.351 type);
one or more (for example, 1, 2 or 3) selected from the group consisting of substitution of the 417th amino acid residue, lysine (K417) or an amino acid residue corresponding thereto with another amino acid (e.g., K417T), substitution of the 484th amino acid residue, glutamic acid (E484) or an amino acid residue corresponding thereto with another amino acid (e.g., E484K), and substitution of the 501th amino acid residue, asparagine (N501) or an amino acid residue corresponding thereto with another amino acid (e.g., N501Y) (for example, Brazil mutation SARS-CoV-2 virus, P.1 type);
substitution of the 452th amino acid residue, leucine (L452) or an amino acid residue corresponding thereto with another amino acid (e.g., L452R) (for example, California mutation SARS-CoV-2 virus, B.1.427 type/B.1.429 type); or
one or more (for example, 1 or 2) selected from the group consisting of substitution of the 477th amino acid residue, serine (S477) or an amino acid residue corresponding thereto with another amino acid (e.g., S477N), and substitution of the 484th amino acid residue, glutamic acid (E484) or an amino acid residue corresponding thereto with another amino acid (e.g., E484K) (for example, New York mutation SARS-CoV-2 virus, B.1.526 type),
wherein the amino acid position is counted from the N-terminus based on the spike protein of SEQ ID NO: 1.

In one specific embodiment, the mutation may be substitution of at least one (for example, 1, 2, 3, 4 or 5) selected from the group consisting of:
the 417th amino acid residue, lysine (K417), or an amino acid residue corresponding thereto (the 99th amino acid residue from the N-terminus based on SEQ ID NO: 2 (wild-type SARS-CoV-2 RBD)),
the 452th amino acid residue, leucine (L452), or an amino acid residue corresponding thereto (the 134th amino acid residue from the N-terminus based on SEQ ID NO: 2),
the 477th amino acid residue, serine (S477), or an amino acid residue corresponding thereto (the 159th amino acid residue from the N-terminus based on SEQ ID NO: 2),
the 484th amino acid residue, glutamic acid (E484), or an amino acid residue corresponding thereto (the 166th amino acid residue from the N-terminus based on SEQ ID NO: 2), and
the 501th amino acid residue, asparagine (N501) or an amino acid residue corresponding thereto (the 183th amino acid residue from the N-terminus based on SEQ ID NO: 2),
with another amino acid,
wherein the amino acid position is counted from the N-terminus based SEQ ID NO: 1 (spike protein).

In an embodiment, the mutation may comprise at least one (for example, 1, 2, 3, 4 or 5) selected from the group consisting of:
substitution of the 417th amino acid residue, lysine, with threonine (K417T) or with asparagine (K417N), substitution of the 452th amino acid residue, leucine (L452), with arginine (L452R), substitution of the 477th amino acid residue, serine (S477), with asparagine (S477N), substitution of the 484th amino acid residue, glutamic acid, with lysine (E484K), and substitution of the 501th amino acid residue, asparagine, with tyrosine (N501Y), based SEQ ID NO: 1, but not limited thereto.

The wild-type and mutant amino acid sequences of the SARS-CoV-2 RBD are illustrated in Table 1 below:

**[Table 1]**

| Amino acid sequence of SARS-CoV-2 RBD | | |
|---|---|---|
| | Amino acid sequence (N-terminus→C-terminus) | SEQ ID NO: |
| RBD_Origin al | RVQPTESIVRFPNITNLCPFGEVFNATRFASVYAWN RKRISNCVADYSVLYNSASFSTFKCYGVSPTKLNDL CFTNVYADSFVIRGDEVRQIAPGQTGKIADYNYKLP DDFTGCVIAWNSNNLDSKVGGNYNYLYRLFRKSNL KPFERDISTEIYQAGSTPCNGVEGFNCYFPLQSYGF QPTNGVGYQPYRVVVLSFELLHAPATVCGPKKSTN LVKNKCVNF | 2 |
| RBD_BR(K 417T, E484K, N501Y; P.1 type) | RVQPTESIVRFPNITNLCPFGEVFNATRFASVYAWN RKRISNCVADYSVLYNSASFSTFKCYGVSPTKLNDL CFTNVYADSFVIRGDEVRQIAPGQTGTIADYNYKLPD DFTGCVIAWNSNNLDSKVGGNYNYLYRLFRKSNLK PFERDISTEIYQAGSTPCNGVKGFNCYFPLQSYGFQ PTYGVGYQPYRVVVLSFELLHAPATVCGPKKSTNLV KNKCVNF | 3 |
| RBD_ZA(K4 17N, E484K, N501Y; B.1.351 type) | RVQPTESIVRFPNITNLCPFGEVFNATRFASVYAWN RKRISNCVADYSVLYNSASFSTFKCYGVSPTKLNDL CFTNVYADSFVIRGDEVRQIAPGQTGNIADYNYKLP DDFTGCVIAWNSNNLDSKVGGNYNYLYRLFRKSNL KPFERDISTEIYQAGSTPCNGVKGFNCYFPLQSYGF QPTYGVGYQPYRVVVLSFELLHAPATVCGPKKSTN LVKNKCVNF | 4 |
| RBD_CA (L452R, B.1.427/B.1. 429) | RVQPTESIVRFPNITNLCPFGEVFNATRFASVYAWN RKRISNCVADYSVLYNSASFSTFKCYGVSPTKLNDL CFTNVYADSFVIRGDEVRQIAPGQTGKIADYNYKLP DDFTGCVIAWNSNNLDSKVGGNYNYRYRLFRKSNL KPFERDISTEIYQAGSTPCNGVEGFNCYFPLQSYGF QPTNGVGYQPYRVVVLSFELLHAPATVCGPKKSTN LVKNKCVNF | 5 |
| RBD_NY (S477N, E484K, B.1.526) | RVQPTESIVRFPNITNLCPFGEVFNATRFASVYAWN RKRISNCVADYSVLYNSASFSTFKCYGVSPTKLNDL CFTNVYADSFVIRGDEVRQIAPGQTGKIADYNYKLP DDFTGCVIAWNSNNLDSKVGGNYNYLYRLFRKSNL KPFERDISTEIYQAGNTPCNGVKGFNCYFPLQSYGF QPTNGVGYQPYRVVVLSFELLHAPATVCGPKKSTN LVKNKCVNF | 6 |
| RBD_Origin al_fragment | CPFGEVFNATRFASVYAWNRKRISNCVADYSVLYN SASFSTFKCYGVSPTKLNDLCFTNVYADSFVIRGDE VRQIAPGQTGKIADYNYKLPDDFTGCVIAWNSNNLD SKVGGNYNYLYRLFRKSNLKPFERDISTEIYQAGST PCNGVEGFNCYFPLQSYGFQPTNGVGYQPYRVVVL SFELLHAPATVC | 7 |
| RBD_BR(K 417T, E484K, N501Y) _fragment | CPFGEVFNATRFASVYAWNRKRISNCVADYSVLYN SASFSTFKCYGVSPTKLNDLCFTNVYADSFVIRGDE VRQIAPGQTGTIADYNYKLPDDFTGCVIAWNSNNLD SKVGGNYNYLYRLFRKSNLKPFERDISTEIYQAGST PCNGVKGFNCYFPLQSYGFQPTYGVGYQPYRVVVL SFELLHAPATVC | 8 |
| RBD_ZA(K4 17N, E484K, N501Y) _fragment | CPFGEVFNATRFASVYAWNRKRISNCVADYSVLYN SASFSTFKCYGVSPTKLNDLCFTNVYADSFVIRGDE VRQIAPGQTGNIADYNYKLPDDFTGCVIAWNSNNLD SKVGGNYNYLYRLFRKSNLKPFERDISTEIYQAGST PCNGVKGFNCYFPLQSYGFQPTYGVGYQPYRVVVL SFELLHAPATVC | 9 |
| RBD_CA (L452R)_fra gment | CPFGEVFNATRFASVYAWNRKRISNCVADYSVLYN SASFSTFKCYGVSPTKLNDLCFTNVYADSFVIRGDE VRQIAPGQTGKIADYNYKLPDDFTGCVIAWNSNNLD SKVGGNYNYRYRLFRKSNLKPFERDISTEIYQAGST PCNGVEGFNCYFPLQSYGFQPTNGVGYQPYRVVVL SFELLHAPATVC | 10 |
| RBD_NY (S477N, E484K)_fra gment | CPFGEVFNATRFASVYAWNRKRISNCVADYSVLYN SASFSTFKCYGVSPTKLNDLCFTNVYADSFVIRGDE VRQIAPGQTGKIADYNYKLPDDFTGCVIAWNSNNLD SKVGGNYNYLYRLFRKSNLKPFERDISTEIYQAGNT PCNGVKGFNCYFPLQSYGFQPTNGVGYQPYRVVVL SFELLHAPATVC | 11 |
| Spike protein; GenBank: QHD43416. 1 (RBD: 319..541) | | 1 |
| | | |

| | | |
|---|---|---|
| (Bold: RBD; Italic letter: polypeptide fragment of RBD; Underline: mutant amino acid residue) | | |

The SARS-CoV-2 RBD comprised in the conjugate provided in the present description may be selected from SEQ ID NOs: 2 to 11, but not limited thereto.

The hepatitis B surface antigen is a C-terminal fragment of hepatitis B virus (HBV) middle S Protein (UniprotKB: DOESRO) (SEQ ID NO: 14), and may be used for preparation of a vaccine for prevention of hepatitis in a VLP (Virus-Like Particle) form, as expressed as a recombinant protein in an appropriate host cell (for example, animal cells including yeast, E. coli, CHO, and the like). In one embodiment, the hepatitis B surface antigen may be selected from SEQ ID NOs: 12 to 14 (Table 2).

**[Table 2]**

| Amino acid sequence of hepatitis B virus surface antigen | | |
|---|---|---|
| | Amino acid sequence (N-terminus→C-terminus) | SEQ ID NO: |
| HBsAg1 | MESTTSGFLGPLLVLQAGFFLLTRILTIPQSLDSWWTSL NFLGGAPTCPGQNSQSPTSNHSPTSCPPICPGYRWMC LRRFIIFLFILLLCLIFLLVLLDYQGMLPVCPLLPGTSTTST GPCKTCTIPAQGTSMFPSCCCTKPSDGNCTCIPIPSSWA FARFLWEWASVRFSWLSLLVPFVQWFAGLSPTVWLSVI WMMWYWGPSLYNILSPFLPLLPIFFCLWVYI | 12 |
| HBsAg2 | PAPNMESTTSGFLGPLLVLQAGFFLLTRILTIPQSLDSW WTSLNFLGGAPTCPGQNSQSPTSNHSPTSCPPICPGYR WMCLRRFIIFLFILLLCLIFLLVLLDYQGMLPVCPLLPGTS TTSTGPCKTCTIPAQGTSMFPSCCCTKPSDGNCTCIPIP SSWAFARFLWEWASVRFSWLSLLVPFVQWFAGLSPTV WLSVIWMMWYWGPSLYNILSPFLPLLPIFFCLWVYI | 13 |
| HBV middle S Protein (UniprotK B: DOESRO) | MQWNSTTFHQALLDPRVRGLYFPAGGSSSGTVNPVPTT ASPISSIFSRTGDPAPNMESTTSGFLGPLLVLQAGFFLLT RILTIPQSLDSWWTSLNFLGGAPTCPGQNSQSPTSNHS PTSCPPICPGYRWMCLRRFIIFLFILLLCLIFLLVLLDYQG MLPVCPLLPGTSTTSTGPCKTCTIPAQGTSMFPSCCCTK PSDGNCTCIPIPSSWAFARFLWEWASVRFSWLSLLVPF VQWFAGLSPTVWLSVIWMMWYWGPSLYNILSPFLPLLP IFFCLWVYI | 14 |

The conjugate provided in the present description, may be obtained by mixing the aforementioned hepatitis B virus surface antigen and SARS-CoV-2 RBD at a molar ratio of 1:0.1 to 100, 1:0.1 to 70, 1:0.1 to 50, 1:0.1 to 30, 1:0.1 to 10, 1:0.1 to 7, 1:0.1 to 5, 1:0.1 to 3.5, 1:0.1 to 3, 1:0.1 to 2.5, 1:0.1 to 2.1, 1:0.1 to 2, 1:0.1 to 1.5, 1:0.1 to 1, 1:0.1 to 0.7, 1:0.1 to 0.5, 1:0.3 to 100, 1:0.3 to 70, 1:0.3 to 50, 1:0.3 to 30, 1:0.3 to 10, 1:0.3 to 7, 1:0.3 to 5, 1:0.3 to 3.5, 1:0.3 to 3, 1:0.3 to 2.5, 1:0.3 to 2.1, 1:0.3 to 2, 1:0.3 to 1.5, 1:0.3 to 1, 1:0.3 to 0.7, 1:0.3 to 0.5, 1:0.5 to 100, 1:0.5 to 70, 1:0.5 to 50, 1:0.5 to 30, 1:0.5 to 10, 1:0.5 to 7, 1:0.5 to 5, 1:0.5 to 3.5, 1:0.5 to 3, 1:0.5 to 2.5, 1:0.5 to 2.1, 1:0.5 to 2, 1:0.5 to 1.5, 1:0.5 to 1, 1:0.5 to 0.7, 1:0.7 to 100, 1:0.7 to 70, 1:0.7 to 50, 1:0.7 to 30, 1:0.7 to 10, 1:0.7 to 7, 1:0.7 to 5, 1:0.7 to 3.5, 1:0.7 to 3, 1:0.7 to 2.5, 1:0.7 to 2.1, 1:0.7 to 2, 1:0.7 to 1.5, 1:0.7 to 1, 1:1 to 100, 1:1 to 70, 1:1 to 50, 1:1 to 30, 1:1 to 10, 1:1 to 7, 1:1 to 5, 1:1 to 3.5, 1:1 to 3, 1:1 to 2.5, 1:1 to 2.1, 1:1 to 2, 1:1 to 1.5, 1:0.1 to 1, 1:0.1 to 0.7, or 1:0.1 to 0.5 (mole of hepatitis B virus surface antigen : mole of SARS-CoV-2 RBD), but not limited thereto.

The linker may be selected from crosslinking agent compounds having a functional group capable of linking a protein, and in one embodiment, the chemical linker may be a compound having a functional group comprising oxygen, nitrogen or sulfur, for example, one or more kinds of functional groups selected from the group consisting of carbonyl, imide, ester, thiol, sulfonyl, and the like, and for example, it may be one or more kinds selected from the group consisting of glutaraldehyde, SMPH (succinimidyl-6-((b-maleimidopropionamido)hexanoate), SMPB (Succinimidyl 4-(p-maleimido-phenyl)butyrate), Sulfo-SMPB, Sulfo-EMCS [Sulfo-(N-ε-maleimidocaproyl-oxysulfosuccinimide ester)], Sulfo-GMBS [Sulfo-(N-γ-maleimidobutyryl-oxysuccinimide ester)], SM[PEG]n (NHS-PEGn-Maliemide) , AMAS (N-(α-Maleimidoacetoxy)-succinimide ester), BMPS (N-(β-Maleimidopropyloxy)succinimide ester), EMCA (N-ε-Maleimidocaproic acid), EMCS (N-(ε-Maleimidocaproyloxy)succinimide ester), GMBS (N-(γ-Maleimidobutyryloxy)succinimide ester), LC-SMCC (Succinimidyl 4-(N-maleimidomethyl) cyclohexane-1-carboxy-(6-amidocaproate)), MBS (m-Maleimidobenzoyl-N-hydroxysuccinimide ester), SBAP (Succinimdyl 3-(bromoacetamido)propionate), SIA (N-succinimidyl iodoacetate), SIAB (N-Succinimidyl(4-iodoacetyl)aminobenzoate), Sulfo-KMUS (N-(κ-Maleimidoundecanoyloxy)sulfosuccinimide ester), Sulfo-MBS (m-Maleimidobenzoyl-N-hydroxysulfosuccinimide ester), SUlfo-SIAB (Sulfosuccinimidyl(4-iodo-acetyl)aminobenzoate), and the like, but not limited thereto.

In one specific embodiment, the glutaraldehyde is a crosslinking agent comprising two aldehyde groups as a functional group, and may form an amide bond (peptide bond) by reacting with an amino group of a protein. The amino group forming an amide bond with the glutaraldehyde may be an amino group at the N-terminus of a protein (NH2-) or an amino group of lysine in a protein. For example, one aldehyde group of glutaraldehyde may bind to the amino group at the N-terminus of the hepatitis B virus surface antigen or amino group of lysine positioned in the middle, and the other aldehyde group may bind to the amino group at the N-terminus of the SARS-CoV-2 RBD or amino group of lysine positioned in the middle of the amino acid sequence, thereby linking the SARS-CoV-2 RBD and hepatitis B virus surface antigen. In the vaccine composition provided in the present description, the glutaraldehyde as the linker may be comprised or used (added) in an amount of about 0.04 to about 1 %(w/v), about 0.04 to about 0.8 %(w/v), about 0.04 to about 0.5 %(w/v), about 0.04 to about 0.3 %(w/v), about 0.04 to about 0.1 %(w/v), about 0.06 to about 1 %(w/v), about 0.06 to about 0.8 %(w/v), about 0.06 to about 0.5 %(w/v), about 0.06 to about 0.3 %(w/v), about 0.06 to about 0.1 %(w/v), about 0.08 to about 1 %(w/v), about 0.08 to about 0.8 %(w/v), about 0.08 to about 0.5 %(w/v), about 0.08 to about 0.3 %(w/v), about 0.08 to about 0.1 %(w/v), about 0.1 to about 1 %(w/v), about 0.1 to about 0.8 %(w/v), about 0.1 to about 0.5 %(w/v), or about 0.1 to about 0.3 %(w/v), but not limited thereto.

In another specific embodiment, the SMPH is a bifunctional cross-linker comprising an NHS ester group and a maleimide group as a functional group. The SMPH may link lysine positioned in the middle of an amino acid sequence of one protein and free cysteine of another protein. In one embodiment, the SMPH may link lysine positioned in the middle of the amino acid sequence of the hepatitis B virus surface antigen and free cysteine of the SARS-CoV-2 RBD. In the vaccine composition provided in the present description, the SMPH as the linker, may be comprised or used (added) in an amount of 2 to 40 moles, 2 to 35 moles, 2 to 30 moles, 2 to 25 moles, 2 to 20 moles, 5 to 40 moles, 5 to 35 moles, 5 to 30 moles, 5 to 25 moles, 5 to 20 moles, 7 to 40 moles, 7 to 35 moles, 7 to 30 moles, 7 to 25 moles, 7 to 20 moles, 10 to 40 moles, 10 to 35 moles, 10 to 30 moles, 10 to 25 moles, or 10 to 20 moles, based on the 1 mole of the hepatitis B virus surface antigen, but not limited thereto.

### Virus-like particle

In the conjugate, the SARS-CoV-2 RBD may be linked to the hepatitis B virus surface antigen in a virus-like particle (VLP) form through or without a linker, or a VLP form in which the hepatitis B virus surface antigen, in which the SARS-CoV-2 RBD is linked through or without a linker, self-assembles and the SARS-CoV-2 RBD is positioned (exposed) on the particle surface may be formed. Therefore, another embodiment provides a virus-like particle comprising the SARS-CoV-2 RBD-HBsAg conjugate.

In one specific embodiment, the SARS-CoV-2 RBD and hepatitis B virus surface antigen may be each expressed in a suitable host cell (for example, mammal cells such as yeast, E. coli, CHO, etc.), respectively, or an RBD encoding gene of the SARS-CoV-2 and a hepatitis B virus surface antigen encoding gene may be expressed, thereby preparing/purifying it as a VLP.

For example, in the virus-like particle,
the hepatitis B virus surface antigen exposed to the surface of the virus-like particle in which the hepatitis B virus surface antigen is formed (self-assembles) and the RBD of the SARS-CoV-2 may be linked through or without a linker, or
it may be formed (self-assemble) by the hepatitis B virus surface antigens among conjugates formed by linking the hepatitis B virus surface antigen and RBD of the SARS-CoV-2 through or without a linker.

The virus-like particle is a very similar molecule to a virus, but it does not have a virus genetic material, and therefore, it is not contagious. The hepatitis B virus surface antigen may form a virus-like particle by self-assembly.

### Medical use of vaccine composition and the like

Other embodiment provides a vaccine composition against coronavirus, comprising the conjugate and/or virus-like particle. The vaccine composition may further comprise an adjuvant.

Other embodiment provides a method for immunization against coronavirus, comprising administering the conjugate and/or virus-like particle, or vaccine composition into a subject in need of immunization against coronavirus.

Other embodiment provides a pharmaceutical composition for prevention of infection or disease and/or symptoms related to infection of coronavirus, comprising the conjugate and/or virus-like particle, or vaccine composition.

Other embodiment provides a method for prevention of infection or disease and/or symptoms related to infection of coronavirus, comprising administering the conjugate and/or virus-like particle, or vaccine composition into a subject in need of prevention of infection or disease and/or symptoms related to infection of coronavirus.

Other embodiment provides a pharmaceutical composition for treatment of infection or disease and/or symptoms related to infection of coronavirus, comprising an antibody binding to the conjugate and/or virus-like particle, or blood or serum isolated from a subject administered with the conjugate and/or virus-like particle. The antibody may be isolated from blood or serum isolated from a subject administered with the conjugate and/or virus-like particle.

Other embodiment provides a method for treatment of infection or disease and/or symptoms related to infection of coronavirus, comprising administering an antibody binding to the conjugate and/or virus-like particle, or blood or serum isolated from a subject administered with the conjugate and/or virus-like particle into a patient in need of treatment of infection or disease and/or symptoms related to infection of coronavirus. The antibody may be isolated from blood or serum isolated from a subject administered with the conjugate and/or virus-like particle.

The coronavirus may be SARS-CoV-2 virus.

The content of the conjugate and/or virus-like particle comprised as an antigen in the vaccine composition may be 0.0001 mg/mL to 1 mg/mL, 0.0001 mg/mL to 0.9 mg/mL, 0.0001 mg/mL to 0.8 mg/mL, 0.0001 mg/mL to 0.7 mg/mL, 0.0001 mg/mL to 0.6 mg/mL, 0.0001 mg/mL to 0.5 mg/mL, 0.0001 mg/mL to 0.4 mg/mL, 0.0001 mg/mL to 0.3 mg/mL, 0.0001 mg/mL to 0.2 mg/mL, 0.0005 mg/mL to 1 mg/mL, 0.0005 mg/mL to 0.9 mg/mL, 0.0005 mg/mL to 0.8 mg/mL, 0.0005 mg/mL to 0.7 mg/mL, 0.0005 mg/mL to 0.6 mg/mL, 0.0005 mg/mL to 0.5 mg/mL, 0.0005 mg/mL to 0.4 mg/mL, 0.0005 mg/mL to 0.3 mg/mL, 0.0005 mg/mL to 0.2 mg/mL, 0.001 mg/mL to 1 mg/mL, 0.001 mg/mL to 0.9 mg/mL, 0.001 mg/mL to 0.8 mg/mL, 0.001 mg/mL to 0.7 mg/mL, 0.001 mg/mL to 0.6 mg/mL, 0.001 mg/mL to 0.5 mg/mL, 0.001 mg/mL to 0.4 mg/mL, 0.001 mg/mL to 0.3 mg/mL, 0.001 mg/mL to 0.2 mg/mL, 0.004 mg/mL to 1 mg/mL, 0.004 mg/mL to 0.9 mg/mL, 0.004 mg/mL to 0.8 mg/mL, 0.004 mg/mL to 0.7 mg/mL, 0.004 mg/mL to 0.6 mg/mL, 0.004 mg/mL to 0.5 mg/mL, 0.004 mg/mL to 0.4 mg/mL, 0.004 mg/mL to 0.3 mg/mL, 0.004 mg/mL to 0.2 mg/mL, 0.008 mg/mL to 1 mg/mL, 0.008 mg/mL to 0.9 mg/mL, 0.008 mg/mL to 0.8 mg/mL, 0.008 mg/mL to 0.7 mg/mL, 0.008 mg/mL to 0.6 mg/mL, 0.008 mg/mL to 0.5 mg/mL, 0.008 mg/mL to 0.4 mg/mL, 0.008 mg/mL to 0.3 mg/mL, 0.008 mg/mL to 0.2 mg/mL, 0.01 mg/mL to 1 mg/mL, 0.01 mg/mL to 0.9 mg/mL, 0.01 mg/mL to 0.8 mg/mL, 0.01 mg/mL to 0.7 mg/mL, 0.01 mg/mL to 0.6 mg/mL, 0.01 mg/mL to 0.5 mg/mL, 0.01 mg/mL to 0.4 mg/mL, 0.01 mg/mL to 0.3 mg/mL, 0.01 mg/mL to 0.2 mg/mL, 0.05 mg/mL to 1 mg/mL, 0.05 mg/mL to 0.9 mg/mL, 0.05 mg/mL to 0.8 mg/mL, 0.05 mg/mL to 0.7 mg/mL, 0.05 mg/mL to 0.6 mg/mL, 0.05 mg/mL to 0.5 mg/mL, 0.05 mg/mL to 0.4 mg/mL, 0.05 mg/mL to 0.3 mg/mL, 0.05 mg/mL to 0.2 mg/mL, 0.1 mg/mL to 1 mg/mL, 0.1 mg/mL to 0.9 mg/mL, 0.1 mg/mL to 0.8 mg/mL, 0.1 mg/mL to 0.7 mg/mL, 0.1 mg/mL to 0.6 mg/mL, 0.1 mg/mL to 0.5 mg/mL, 0.1 mg/mL to 0.4 mg/mL, 0.1 mg/mL to 0.3 mg/mL, or 0.1 mg/mL to 0.2 mg/mL, but not limited thereto.

The coronavirus vaccine composition may further comprise one or more selected from the group consisting of a potentiator, a preservative, a buffer, a surfactant, a carrier, an osmagent (tonicity agent), an antioxidant, and a stabilizer.

In the present description, the term "adjuvant (or potentiator or immunopotentiator)" refers to a substance used for increasing immunogenicity of the immunogenic composition of the present invention. The adjuvant is often provided for enhancing an immune response, and this is well known to those skilled in the art.

In the present invention, the term "preservative" refers to a substance having an anti-viral and/or antibacterial action that inhibits proliferation of a microorganism in the vaccine composition, and for example, it may be one or more kinds (one kind, 2 kinds or 3 kinds) selected from the group consisting of thimerosal, 2-phenoxyethanol, formaldehyde, and the like, but not limited thereto, and all common preservatives used in the art may be used. For example, the preservative may comprise phenoxyethanol (2-phenoxyethanol), formaldehyde, or a mixture thereof, and in this case,

the content of the phenoxyethanol in the vaccine composition may be 0.5 mg/mL to 20 mg/mL, 0.5 mg/mL to 18 mg/mL, 0.5 mg/mL to 15 mg/mL, 0.5 mg/mL to 12 mg/mL, 0.5 mg/mL to 10 mg/mL, 1 mg/mL to 20 mg/mL, 1 mg/mL to 18 mg/mL, 1 mg/mL to 15 mg/mL, 1 mg/mL to 12 mg/mL, 1 mg/mL to 10 mg/mL, 3 mg/mL to 20 mg/mL, 3 mg/mL to 18 mg/mL, 3 mg/mL to 15 mg/mL, 3 mg/mL to 12 mg/mL, 3 mg/mL to 10 mg/mL, 3.75 mg/mL to 20 mg/mL, 3.75 mg/mL to 18 mg/mL, 3.75 mg/mL to 15 mg/mL, 3.75 mg/mL to 12 mg/mL, or 3.75 mg/mL to 10 mg/mL, 4 mg/mL to 20 mg/mL, 4 mg/mL to 18 mg/mL, 4 mg/mL to 15 mg/mL, 4 mg/mL to 12 mg/mL, 4 mg/mL to 10 mg/mL, 4 mg/mL to 8 mg/mL, or 4 mg/mL to 6 mg/mL, based on the total vaccine composition.

The content of the formaldehyde in the vaccine composition may be 1ug/mL to 200ug/mL, 1ug/mL to 180ug/mL, 1ug/mL to 150ug/mL, 1ug/mL to 120ug/mL, 1ug/mL to 100ug/mL, or 1ug/mL to 80ug/mL, based on the total vaccine composition.

In addition, the vaccine composition may comprise one or more kinds of, physiologically acceptable buffers. For example, when the vaccine composition is infusion or injection, the buffer may have buffer capacity at pH 4.0 to 10.0, specifically, pH 5.0 to 9.0, more specifically, pH 6.0 to 8.0. The buffer may be one or more kinds selected from the group consisting of TRIS, acetate, glutamate, lactate, maleate, tartrate, phosphate, citrate, carbonate, glycinate, histidine, glycine, succinate, and triethanolamine buffer.

In particular, when the vaccine composition of the present invention is on purpose of parenteral administration, the buffer may be selected from buffers suitable for USP. For example, the buffer may be one or more kinds selected from the group consisting of monobasic acids such as acetic acid, benzoic acid, gluconic acid, glucaric acid and lactic acid; dibasic acids such as aconitinic acid, adipic acid, ascorbic acid, carbonic, glutamic acid, malic acid, succinic acid and tartaric acid; polybasic acids such as citric acid and phosphoric acid; and bases such as ammonia, diethanolamine, glycine, triethanolamine and TRIS.

Furthermore, the vaccine composition provided in the present invention may further comprise a non-ionic surfactant. The example of the non-ionic surfactant which can be further comprised as such, may include one or more kinds selected from the group consisting of polyoxyethylene sorbitan ester compounds (polysorbate 20 and polysorbate 80), copolymers of ethylene oxide (EO), propylene oxide (PO), butylene oxide (BO) (e.g.: DOWFAXTM); oxtoxynols having different repeating numbers of an oxy-1,2-ethanediyl group, particularly, oxtoxynol-9 (Triton-100); ethylphenoxypolyethoxyethanol (IGEPAL CA-630/NP-40); phospholipids such as lecithin; nonylphenol ethoxylate such as NP series; polyoxyethylene fatty acid ether induced in lauryl, cetyl, stearyl, oleyl alcohol (Brij surfactant), particularly, triethyleneglycol monolauryl ether (Brij 30); sorbitan ether known as SPANs, particularly, sorbitan trioleate (Span 85) and sorbitan monolaurate and the like, but not limited thereto. Specifically, the polyoxyethylene sorbitan ester compounds (polysorbate 20 and polysorbate 80) may be comprised in an amount of 0.001 %(w/v) to 20 %(w/v), for example, 0.001 %(w/v) to 10 %(w/v), particularly, 0.001 %(w/v) to 1 %(w/v) or about 0.5 %(w/v), but not limited thereto.

The physiologically acceptable carrier used for a liquid preparation includes an aqueous or non-aqueous solvent, suspension, emulsion and oil. The example of the non-aqueous solvent includes propylene glycol, polyethylene glycol and ethyl oleate. The aqueous carrier includes water, alcohol/aqueous solvent, emulsion or suspension, physiological saline solution, and buffer solution. The example of oil includes vegetable or animal oil, peanut oil, soybean oil, olive oil, sunflower oil, liver oil, synthetic oil such as marine oil, and lipid obtained from milk or eggs. The vaccine composition of the present invention may be isotonic, hypertonic or hypotonic, and the pharmaceutical composition administered by infusion or injection is preferably isotonic fundamentally, but not limited thereto. On the other hand, isotonicity or hypertonicity may be advantageous for storage of the composition. When the vaccine composition is hypertonic, it may be diluted so as to be isotonic before administration. A tonicity agent for dilution may be an ionic tonicity agent such as a salt, or a non-ionic tonicity agent such as a carbohydrate. The ionic tonicity agent includes sodium chloride, calcium chloride, potassium chloride, magnesium chloride, and the like, but not limited thereto. The non-ionic tonicity agent includes sorbitol, glycerol and the like, but not limited thereto.

The vaccine composition provided in the present description may comprise an antioxidant. An exemplary antioxidant includes mercaptopropionyl glycine, N acetylcysteine, β-mercaptoethylamine, glutathione, ascorbic acid and salts thereof, sulfite or sodium methabisulfite or similar species. Additionally, the antioxidant may also include a natural antioxidant such as vitamins E and C, lecithin, xanthine and beta carotene, and a substance such as zinc and selenium, but not limited thereto.

The vaccine composition provided in the present description may comprise a stabilizer, a wetting agent, an emulsifier, a dispersing agent and an additional substance such as a monosaccharide, a polysaccharide, and the like. In addition, it may comprise an immunostimulatory molecule to enhance vaccine efficacy. This molecule may intensify an immune response, and cause inflammation, and it may be a specific lymphocyte or cytokine. The cytokine may include interleukin (IL)-1, IL-2, IL-3, IL4, IL-12, IL-13, granulocyte-macrophage colony-stimulating factor (GMCSF), macrophage inflammation factor, and the like, but not limited thereto.

The coronavirus vaccine composition may be a composition formulated as a single dose for single administration or a multiple dose (multidose) for multiple administration. As used in the present description, "multiple dose" may mean a preparation unit comprising a vaccine dose that can be administered (inoculated) more than once (for example, two or more times) into a 1 administered (inoculated) subject, or a vaccine dose that can be administered (inoculated) once or more than once (for example, two or more times) into a 2 or more administered (inoculated) subject.

The optimum amount of a component for a specific vaccine may be confirmed by standard research including observation of an appropriate immune response in a subject. For example, by extrapolating the animal experiment result, a vaccination dose for a human may be determined. In addition, those skilled in the art can determine the dose empirically, according to need.

The vaccine composition may further comprise an adjuvant. The adjuvant available in the present description may be one or more kinds selected from all pharmaceutically acceptable components capable of intensifying an immune response (for example, neutralizing antibody titer, binding antibody titer, etc.) of an inoculated person of the vaccine.

For example, the adjuvant may be one or more kinds selected from the group consisting of
(1) aluminum ion containing adjuvants including aluminum hydroxide (Al(OH)₃), aluminum phosphate (AlPO₄), AAHS (amorphous aluminum hydroxyphosphate sulfate), Alum and the like (Alhydrogel^{®}, Rehydragel^{™}, Adju-Phos^{®}, etc.);
(2) calcium ion containing adjuvants including calcium phosphate (CaPO₄) and the like;
(3) biocompatible oil-based (containing) emulsion adjuvants such as squalene, cholesterol, mineral oil (paraffin, etc.), and the like (oil-in-water or water-in-oil emulsion; ASO3, MF59, AddaS03^{™}, AddaVax^{™}, ISCOMATRIXO, Freund's Adjuvant, etc.);
(4) liposome containing adjuvants such as virosome and the like, lipid containing adjuvants such as Monophosphoryl lipid A (MPL) and the like (ASO4, AS018, etc.),
(5) adjuvants containing plant extract such as Quillaja sp. plants (Quillaja saponaria Molina, etc.), soybean, Polygala senega, and the like, or plant extract-derived compounds (e.g., saponin, etc.) (AS018, Quil A, Abisco-100, ISCOMATRIXO, Matrix M^{™}, etc.);
(6) nucleic acid containing adjuvants such as CpG motif and the like (CpG 1018, etc.);
(7) adjuvants containing bacteria such as Mycobacterium sp. bacteria or virus (Freund's Adjuvant, etc.);
(8) peptide or protein containing adjuvants such as MDP (Muramyl dipeptide), Flagellin, HSP (heat shock proteins), and the like;
(9) adjuvants containing cytokine such as interferon (e.g., rIFN, etc.), GMCSF, and the like;
(10) biocompatible polymer containing adjuvants such as an anionic polymer (Carbopol, etc.) and the like,
but not limited thereto.

The content of the adjuvant in the vaccine composition provided in the present description may be appropriately adjusted according to the adjuvant type, the manufacturer's prescription, inoculation method, subject (age, body weight, morbidity, etc.), and/or desired effect.

For example, in case of the aluminum ion containing adjuvant, the content in the vaccine composition, may be 0.1 to 10 mg/ml, 0.1 to 7.5 mg/ml, 0.1 to 5 mg/ml, 0.1 to 2.5 mg/ml, 0.1 to 1 mg/ml, 0.3 to 10 mg/ml, 0.3 to 7.5 mg/ml, 0.3 to 5 mg/ml, 0.3 to 2.5 mg/ml, 0.3 to 1 mg/ml, 0.5 to 10 mg/ml, 0.5 to 7.5 mg/ml, 0.5 to 5 mg/ml, 0.5 to 2.5 mg/ml, 0.5 to 1 mg/ml, 0.7 to 10 mg/ml, 0.7 to 7.5 mg/ml, 0.7 to 5 mg/ml, 0.7 to 2.5 mg/ml, 0.7 to 1 mg/ml, 1 to 10 mg/ml, 1 to 7.5 mg/ml, 1 to 5 mg/ml, or 1 to 2.5 mg/ml, based on the aluminum weight, but not limited thereto.

In other embodiment, a biocompatible oil-based (containing) emulsion adjuvant, AS03 or AddaS03 has a composition of 1.8 % (v/v) polysorbate 80, 5 % (v/v) squalene, 5 % (v/v) α-tocophero!, and PBS (NaCl 121 mM, KCl 2.38 mM, Na₂HPO₄ 7.14 mM, KH₂PO₄ 1.3 mM), and based on this (1X), it may be 0.01X to 1X, 0.1X to 0.5X, but not limited thereto, and it may be appropriately adjusted according to the manufacturer's prescription.

Another biocompatible oil-based (containing) emulsion, MF59 or AddaVax^{™} is a submicron emulsion comprising 5% (v/v) squalene oil, 0.5% (w/v) polysorbate 80, 0.5% (w/v) sorbitan trioleate, and 10 mM sodium citrate buffer, and based on this (1X), it may be 0.01X to 1X, 0.1X to 0.5X, but not limited thereto, and it may be appropriately adjusted according to the manufacturer's prescription.

The vaccine composition according to the present invention may be administered in a pharmaceutically effective dose into a subject in need of prevention of occurrence of infection by coronavirus and/or diseases and/or symptoms by coronavirus infection (for example, at risk of occurrence of infection by coronavirus and/or diseases and/or symptoms by coronavirus infection), for prevention of infection by coronavirus and/or diseases and/or symptoms by coronavirus infection.

In the present description, the term "prevention" may refer to any action of inhibiting or delaying occurrence of infection by coronavirus and/or diseases and/or symptoms by coronavirus infection by administration of the vaccine composition of the present invention. The term, "pharmaceutically effective dose" may mean a dose required to induce an antibody sufficient to significantly reduce the probability to be infected by coronavirus or severity of infection. The term "administration" refers to introduction of a prescribed substance into a subject by any suitable method. The vaccine composition may be administered by a route of injection, for example, subcutaneous injection, intravenous injection, intramuscular injection, and the like.

For example, the single dose of the vaccine composition may be about 0.1 to 1000 µg, 0.1 to 900 µg, 0.1 to 800 µg, 0.1 to 700 µg, 0.1 to 600 µg, 0.1 to 500 µg, 0.1 to 400 µg, 0.1 to 350 µg, 0.1 to 300 µg, 0.1 to 250 µg, 0.1 to 200 µg, 0.1 to 150 µg, 0.1 to 100 µg, 0.1 to 80 µg, 0.1 to 60 µg, 0.1 to 40 µg, 0.1 to 20 µg, , 0.5 to 1000 µg, 0.5 to 900 µg, 0.5 to 800 µg, 0.5 to 700 µg, 0.5 to 600 µg, 0.5 to 500 µg, 0.5 to 400 µg, 0.5 to 350 µg, 0.5 to 300 µg, 0.5 to 250 µg, 0.5 to 200 µg, 0.5 to 150 µg, 0.5 to 100 µg, 0.5 to 80 µg, 0.5 to 60 µg, 0.5 to 40 µg, 0.5 to 20 µg, 1 to 1000 µg, 1 to 900 µg, 1 to 800 µg, 1 to 700 µg, 1 to 600 µg, 1 to 500 µg, 1 to 400 µg, 1 to 350 µg, 1 to 300 µg, 1 to 250 µg, 1 to 200 µg, 1 to 150 µg, 1 to 100 µg, 1 to 80 µg, 1 to 60 µg, 1 to 40 µg, 1 to 20 µg, 5 to 1000 µg, 5 to 900 µg, 5 to 800 µg, 5 to 700 µg, 5 to 600 µg, 5 to 500 µg, 5 to 400 µg, 5 to 350 µg, 5 to 300 µg, 5 to 250 µg, 5 to 200 µg, 5 to 150 µg, 5 to 100 µg, 5 to 80 µg, 5 to 60 µg, 5 to 40 µg, 5 to 20 µg, 10 to 1000 µg, 10 to 900 µg, 10 to 800 µg, 10 to 700 µg, 10 to 600 µg, 10 to 500 µg, 10 to 400 µg, 10 to 350 µg, 10 to 300 µg, 10 to 250 µg, 10 to 200 µg, 10 to 150 µg, 10 to 100 µg, 10 to 80 µg, 10 to 60 µg, 10 to 40 µg, or 10 to 20 µg, based on the antigen (the conjugate and/or virus-like particle) weight, but not limited thereto, and it may be appropriately adjusted in consideration of condition of an inoculated person, desired action, and the like.

The "subject" for administration of the conjugate, virus-like particle, vaccine composition or pharmaceutical composition of the present invention may mean a living organism that can be infected with a pathogen or a cell or tissue isolated therefrom or culture thereof, and the organism may be a higher vertebrate, and more specifically, may be a mammal such as a human, but not particularly limited thereto.

### Preparation of conjugate and/or virus-like particle and/or vaccine composition

Other embodiment provides a nucleic acid molecule comprising a combination of a polynucleotide encoding the SARS-CoV-2 RBD and a polynucleotide encoding the hepatitis B virus surface antigen.

Other embodiment provides a recombinant vector comprising the nucleic acid molecule. The recombinant vector may be used as an expression vector in a host cell.

Other embodiment provides a recombinant cell comprising the nucleic acid molecule or recombinant vector. The recombinant cell may be obtained by introducing the recombinant vector to a host cell.

Other embodiment provides a method for preparation of a conjugate comprising the SARS-CoV-2 RBD and hepatitis B virus surface antigen, a virus-like particle comprising the conjugate, or a vaccine composition against coronavirus comprising them, comprising expressing a nucleic acid molecule encoding the SARS-CoV-2 RBD and hepatitis B virus surface antigen in a host cell.

The method may further comprise linking the SARS-CoV-2 RBD and hepatitis B virus surface antigen, by adding a linker to the expressed SARS-CoV-2 RBD and hepatitis B virus surface antigen, after the expressing.

The SARS-CoV-2 RBD and hepatitis B virus surface antigen may be expressed in one host cell or be expressed in separate host cells, respectively, through separate vectors, respectively, and may be produced in a conjugate form through a chemical linker (crosslinking agent).

When the SARS-CoV-2 RBD and hepatitis B virus surface antigen are expressed through separate vectors, respectively,
the hepatitis B virus surface antigen may form a virus-like particle, and the SARS-CoV-2 RBD may form a conjugate and/or may form a virus-like particle comprising the conjugate, as linked with the hepatitis B virus surface antigen on the formed virus-like particle surface through or without a linker; or
the SARS-CoV-2 RBD and hepatitis B virus surface antigen may form a conjugate as linked through or without a linker, and the hepatitis B virus surface antigens in a number of conjugates formed may form a virus-like particle.

In one embodiment, the conjugate or virus-like particle may be obtained by reacting the aforementioned hepatitis B virus surface antigen, SARS-CoV-2 RBD, and linker at the same time, or the conjugate or virus-like particle may be obtained by reacting the aforementioned hepatitis B virus surface antigen, linker and SARS-CoV-2 RBD in order (for example, for the conjugate or virus-like particle, by reacting the aforementioned hepatitis B virus surface antigen and linker first, and then reacting the SARS-CoV-2 RBD). Then, the reaction time may be 10 minutes to 120 minutes, 10 minutes to 90 minutes, 30 minutes to 120 minutes, or 30 minutes to 90 minutes, and the reaction temperature may be 2 to 40 °C, 2 to 35 °C, 2 to 30 °C, 10 to 40 °C, 10 to 35 °C, 10 to 30 °C, 15 to 40 °C, 15 to 35 °C, 15 to 30 °C, 20 to 40 °C, 20 to 35 °C, or 20 to 30 °C, but not limited thereto.

The SARS-CoV-2 RBD, hepatitis B virus surface antigen, linker and ratio and content thereof are as described above.

The host cell may be one or more kinds selected from the group consisting of yeasts (for example, *Saccharomyces cerevisiae, Pichia pastoris,* etc.), E. coli, and animal cells (for example, CHO (Chinese Hamster Ovary) cell, HEK293 cell, Vero cell, Per. C6 cell, etc.), but not limited thereto.

A vector usable as a recombinant vector (expression vector) in the present description is not particularly limited as long as it is capable of replication in a host cell, and may be selected from all vectors commonly used. The example of the commonly used vector may include natural or recombinant plasmids, cosmids, viruses, bacteriophages, and the like. For example, as the phage vector or cosmid vector, pWE15, M13, MBL3, MBL4, IXII, ASHII, APII, t10, t11, Charon4A, and Charon21A and the like may be used, and as the plasmid vector, pBR-based, pUC-based, pBluescriptll-based, pGEM-based, pTZ-based, pCL-based, pCDNA-based and pET-based and the like may be used. Specifically, pDZ, pACYC177, pACYC184, pCL, pECCG117, pUC19, pBR322, pMW118, pCC1BAC, pcDNA3.1 vectors, and the like may be exemplified, but not limited thereto.

The vector may further comprise a selection marker to confirm insertion in the chromosome. The selection marker is for selection transformed cells, that is, confirmation of insertion of the polynucleotide, and may be selected from genes conferring selectable phenotypes such as drug resistance, auxotrophy, resistance to cytotoxic agents or expression of surface proteins, and used. In an environment treated with a selective agent, only the cells expressing the selectable marker survive or exhibit other phenotypic characteristics, so that the transformed cells can be selected.

The protein provided in the present description may not be derived from nature, and may be recombinantly or chemically synthesized. When the protein is recombinantly produced, it may be in a form in which a common signal peptide, a cleavage site, a tag, and the like are bound for purification. Accordingly, in one non-limitative embodiment, the protein provided in the present description may be in a form further comprising one or more selected from the group consisting of a signal peptide, a cleavage site, a tag (for example, His tag, GST (glutathione-s-transferase) tag, MBP (maltose binding protein) tag, etc.) and the like, which can be commonly used for a recombinant production process, or may be in a purified form in which they are removed.

### [ADVANTAGEOUS EFFECTS]

The conjugate provided in the present description or virus-like particle formed therefrom performs an immunogenic action against coronavirus (SARS-CoV-2 (COVID-19)) in vivo, and it may be usefully applied for prevention of coronavirus (SARS-CoV-2 (COVID-19)) infection.

### [BRIEF DESCRIPTION OF THE DRAWINGS]

FIG. 1 is a schematic diagram illustratively showing the process of producing the SARS-CoV-2 RBD-HBsAg VLP conjugate according to one example.
FIG. 2 is a cleavage map of pCB-RBD Vector.
FIG. 3 is the result of purification through IEC (Ion exchange column chromatography) and HIC (Hydrophobic Interaction Chromatography) to confirm SARS-CoV-2 RBD production.
FIG. 4a is a graph showing the DLS (Dynamic Light Scattering) result of the SARS-CoV-2 RBD-HBsAg VLP conjugate (Sample 1) conjugated by using glutaraldehyde (GA) as a linker (crosslinking agent) in comparison with HBsAg VLP.
FIG. 4b is a graph showing the DLS result of the SARS-CoV-2 RBD-HBsAg VLP conjugate (Sample 5) conjugated by using SMPH (succinimidyl-6-((b-maleimidopropionamido)hexanoate) as a linker (crosslinking agent) in comparison with HBsAg VLP.
FIG. 5 is a graph showing the ACE2 binding assay (in vitro) result of the SARS-CoV-2 RBD-HBsAg VLP conjugate produced in the example of the present description.
FIG. 6 is a TEM image of the SARS-CoV-2 RBD-HBsAg VLP conjugate (Sample 4) according to one example.
FIG. 7 is a graph showing the RBD binding antibody titer of the vaccine formulation comprising the SARS-CoV-2 RBD-HBsAg VLP conjugate according to one example.
FIG. 8 is a graph showing the neutralizing antibody titer of the vaccine composition comprising the SARS-CoV-2 RBD-HBsAg VLP conjugate according to one example.
FIG. 9 is a schematic diagram showing the mouse immunization and efficacy test schedule using the vaccine comprising the SARS-CoV-2 RBD-HBsAg VLP conjugate according to one example.
FIG. 10 is a graph showing the neutralizing antibody titer of the vaccine comprising the SARS-CoV-2 RBD-HBsAg VLP conjugate according to one example.

### [MODE FOR INVENTION]

Hereinafter, the present invention will be described in more detail by the following examples. However, they are intended to illustrate the present invention only, but the scope of the present invention is not limited by these examples.

### Example 1: Construction of SARS-CoV-2 RBD-HBsAg VLP conjugate

SARS-CoV-2 (COVID-19) has a spike protein (SEQ ID NO: 1, GenBank: QHD43416) on the virus surface, and an RBD (receptor binding domain; SEQ ID NO: 2, 3, or 4) of the spike protein binds to a receptor of a human cell and enters the inside of the cell, and therefore, the RBD is applicable as an antigen of the SARS-CoV-2 vaccine.

Hepatitis B virus (HBV) middle S Protein (SEQ ID NO: 14, UniprotKB: DOESRO) expresses in a yeast as a recombinant protein, and may be used in a VLP (Virus-Like Particle) form in preparation of a vaccine for preventing hepatitis.

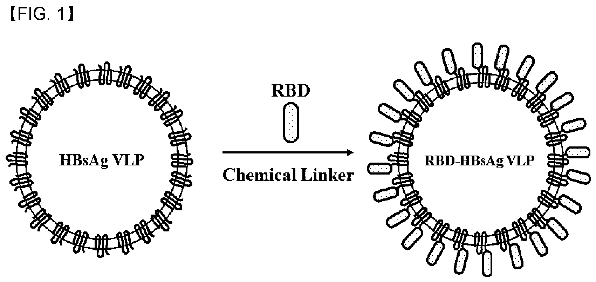

In the present example, by chemical conjugation of an antigen RBD of SARS-CoV-2 (wild-type (SEQ ID NO: 2), mutant (SEQ ID NO: 3, or 4)) to the VLP consisting of antigen HBsAg (SEQ ID NO: 8), which is a C-terminal fragment of HBV middle S Protein, SARS-CoV-2 RBD-HBsAg VLP conjugate is to be constructed (See FIG. 1).

More specifically, by linking a receptor binding domain (RBD; SEQ ID NO: 2), which is the amino acid sequence region from the 319th (R319) to 541th (F541) of the spike protein (Spike protein; GenBank: QHD43416.1; SEQ ID NO: 1) on the SARS-CoV-2 (COVID-19) virus surface, or mutant thereof (SEQ ID NO: 3 or 4) with a hepatitis B surface antigen (HBsAg; SEQ ID NO: 8) through a linker, RBD-HBsAg VLP conjugate was constructed.

The amino acid sequence used for constructing the RBD-HBsAg VLP conjugate were summarized in Table 3 below:

**[Table 3]**

| | Amino acid sequence (N terminus→C terminus) | SEQ ID NO: |
|---|---|---|
| RBD_Origin al | RVQPTESIVRFPNITNLCPFGEVFNATRFASVYAWN RKRISNCVADYSVLYNSASFSTFKCYGVSPTKLNDL CFTNVYADSFVIRGDEVRQIAPGQTGKIADYNYKLP DDFTGCVIAWNSNNLDSKVGGNYNYLYRLFRKSNL KPFERDISTEIYQAGSTPCNGVEGFNCYFPLQSYGF QPTNGVGYQPYRVVVLSFELLHAPATVCGPKKSTN LVKNKCVNF | 2 |
| RBD_BR(K 417T, E484K, N501Y) | RVQPTESIVRFPNITNLCPFGEVFNATRFASVYAWN RKRISNCVADYSVLYNSASFSTFKCYGVSPTKLNDL CFTNVYADSFVIRGDEVRQIAPGQTGTIADYNYKLPD DFTGCVIAWNSNNLDSKVGGNYNYLYRLFRKSNLK PFERDISTEIYQAGSTPCNGVKGFNCYFPLQSYGFQ PTYGVGYQPYRVVVLSFELLHAPATVCGPKKSTNLV KNKCVNF | 3 |
| RBD_ZA(K4 17N, E484K, N501Y) | RVQPTESIVRFPNITNLCPFGEVFNATRFASVYAWN RKRISNCVADYSVLYNSASFSTFKCYGVSPTKLNDL CFTNVYADSFVIRGDEVRQIAPGQTGNIADYNYKLP DDFTGCVIAWNSNNLDSKVGGNYNYLYRLFRKSNL KPFERDISTEIYQAGSTPCNGVKGFNCYFPLQSYGF QPTYGVGYQPYRVVVLSFELLHAPATVCGPKKSTN LVKNKCVNF | 4 |
| HBsAg1 | MESTTSGFLGPLLVLQAGFFLLTRILTIPQSLDSWWT SLNFLGGAPTCPGQNSQSPTSNHSPTSCPPICPGYR WMCLRRFIIFLFILLLCLIFLLVLLDYQGMLPVCPLLP GTSTTSTGPCKTCTIPAQGTSMFPSCCCTKPSDGNC TCIPIPSSWAFARFLWEWASVRFSWLSLLVPFVQW FAGLSPTVWLSVIWMMWYWGPSLYNILSPFLPLLPI FFCLWVYI | 8 |

### 1.1. Preparation of SARS-CoV-2 RBD

The nucleic acid sequence (SEQ ID NO: 15) of the wild-type (original) SARS-CoV-2 RBD (SEQ ID NO: 2) encoding gene (RBD_original) was synthesized and prepared, and using the prepared RBD_original as a template and using the primers RBD2_GS_F and RBD2_R of Table 4 below, RBD PCR Product was obtained. The RBD of the Republic of South Africa (RBD_ZA; SEQ ID NO: 4) was obtained by performing overlap PCR using RBD_Original as a template and using the primers RBD2-GS_F, MutI_ZA_R, Mutl_F, Mutl_R, Mut_II_F, and RBD2_R of Table 4. The RBD of Brazil (RBD_BR) was obtained by performing overlap PCR using RBD_Original as a template and using the primers RBD2-GS_F, MutI_Brazil_R, MutI_F, MutI_R, Mut_II_F, and RBD2_R of Table 4.

**[Table 4]**

| **Name** | **Sequence** | **SEQ ID NO:** | **Memo** |
|---|---|---|---|
| RBD2_GS_ F | agagtccaaccaacagaatc | 16 | RBD2 Cloning |
| RBD2_R | tcttcttcgcggccgctttagaaattgacacatttgtttttaac | 17 | |
| Mutl_F | attgctgattataattataaattac | 18 | RBD2-BR,ZA Cloning |
| Mut_II_F | tcctttacaatcatatggtttccaacccacttatggtg | 19 | |
| MutI_ZA_R | ttataattataatcagcaatgtttc | 20 | |
| MutI_Brazil_ R | ttataattataatcagcaatcgttc | 21 | |
| MutI_R | aaccatatgattgtaaaggaaagtaacaattaaaaccttt aac | 22 | |
| Xslip_F | taaagcggccgcgaagaagaagaag | 23 | pCB Vector Lineariz ation |
| SP_R_RBD 2_OL | gattctgttggttggactcttgcccaggtggggcccccca gga | 24 | |
| RBD_origin al | AGAGTCCAACCAACAGAATCTATTGTTA GATTTCCTAATATTACAAACTTGTGCCC TTTTGGTGAAGTTTTTAACGCCACCAGA TTTGCATCTGTTTATGCTTGGAACAGGA AGAGAATCAGCAACTGTGTTGCTGATT ATTCTGTCCTATATAATTCCGCATCATT TTCCACTTTTAAGTGTTATGGAGTGTCT CCTACTAAATTAAATGATCTCTGCTTTA CTAATGTCTATGCAGATTCATTTGTAAT TAGAGGTGATGAAGTCAGACAAATCGC TCCAGGGCAAACTGGAAAGATTGCTGA TTATAATTATAAATTACCAGATGATTTTA CAGGCTGCGTTATAGCTTGGAATTCTA ACAATCTTGATTCTAAGGTTGGTGGTAA TTATAATTACCTGTATAGATTGTTTAGG AAGTCTAATCTCAAACCTTTTGAGAGAG ATATTTCAACTGAAATCTATCAGGCCG GTAGCACACCTTGTAATGGTGTTGAAG GTTTTAATTGTTACTTTCCTTTACAATCA TATGGTTTCCAACCCACTAATGGTGTT GGTTACCAACCATACAGAGTAGTAGTA CTTTCTTTTGAACTTCTACATGCACCAG CAACTGTTTGTGGACCTAAAAAGTCTA CTAATTTGGTTAAAAACAAATGTGTCAA TTTC | 15 | SARS-CoV-2 RBD (SEQ ID NO: 2) encodin g gene |

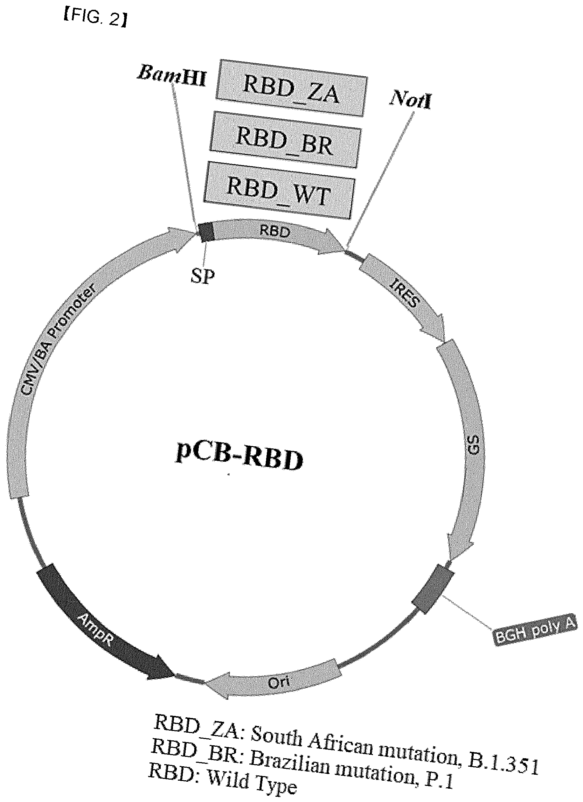

Linearized pCB Vector was obtained by performing PCR using pCB-CVRBD2-His Vector (See FIG. 2) as a template and using the primers Xslip_Fand SP_R_RBD2_OL of Table 4. The pCB Vector has a GS selection marker, and expresses an antigen (RBD) protein by operation of Promoter (KR103836) in which CMV Promoter and CHO beta-actin Promoter are fused, and the antigen protein is secreted outside the cell by operation of Signal Peptide (KR 2092225).

Each of the RBD PCR Products (RBD, RBD_ZA, RBD_BR) obtained as above and Linearized pCB Vector were cloned using EZ Fusion Cloning Kit (Enzynomics, EZ015), and the expression vectors, pCB-RBD, pCB-RBD_ZA, and pCB-RBD_BR were obtained, respectively (See FIG. 2).

The prepared expression vector pCB-RBD (RBD_ZA, RBD_BR) was introduced to CHO (HD-BIOP3, Horizon Discovery), and it was cultured in CD FortiCHO^{™} medium (Thermo Fisher Scientific) for 14 days (Fed-Batch Culture: 3, 5, 7, 9, 11 Feeding), thereby producing the RBD (wild-type, mutant).

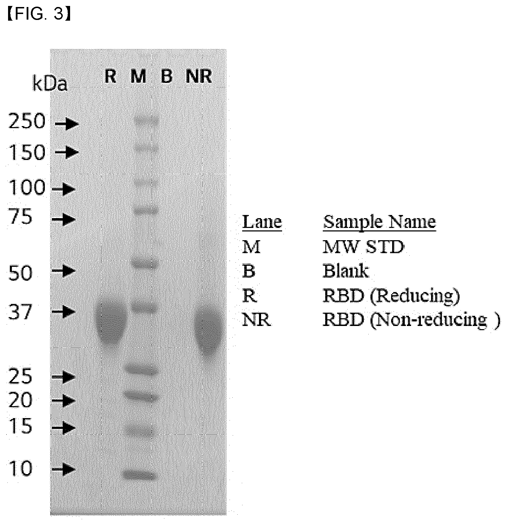

The produced RBD was purified by IEC (Ion exchange column chromatography) and HIC (Hydrophobic Interaction Chromatography) and the like. The obtained purification result was shown in FIG. 3.

### 1.2. Preparation of hepatitis B surface antigen (HBsAg) VLP

The HBsAg VLP was prepared from crude solution in which the antigen HBsAg (SEQ ID NO: 8), a C-terminal fragment of HBV middle S Protein, was expressed in a yeast. The specific method was described in KR0177298, KR0159716, KR0194247, KR0251015 and the like, and the HBsAg constructed by the method disclosed in the document forms HBsAg VLP through Self-Assembly.

### 1.3. Conjugation of SARS-CoV-2 RBD and HBsAg VLP using linker

The SARS-CoV-2 RBD prepared in Example 1.1 and HBsAg VLP prepared in Example 1.2 were conjugated using a linker. Glutaraldehyde (GA) and SMPH (succinimidyl-6-((b-maleimidopropionamido)hexanoate) were used.

Glutaraldehyde (GA) and an amino group are reacted to form an amide bond (=peptide bond). The amino group may be N-term NH₂- of the protein or NH₂ of lysin in the protein. As follows, conjugation using glutaraldehyde was progressed by referring to the following Table 5.

**[Table 5]**

| Sample name | SARS-CoV-2 RBD | HBsAg | Linker | Reaction ratio (HBsAg: RBD) Molar ratio |
|---|---|---|---|---|
| Sample 1 | Wild-type | SEQ ID NO: 8 | GA | 1:1 |
| Sample 2 | Wild-type | SEQ ID NO: 8 | GA | 1:2 |
| Sample A | Wild-type | SEQ ID NO: 8 | GA | 1:3 |

More specifically, the Samples 1 and 2 were prepared by reacting the HBsAg VLP and SARS-CoV-2 RBD under the condition of 0.1%(w/v) Glutaraldehyde and RT (room temperature) for 20 minutes to fit the corresponding conjugation ratio. The Sample A was prepared by reacting the HBsAg VLP under the condition of 0.2% Glutaraldehyde and RT (room temperature) for 60 minutes and then reacting the SARS-CoV-2 RBD for 30 minutes.

SMPH was used as a bi-functional cross-linker for conjugation using lysine of the Yeast-derived HBsAg VLP (Example 1.2) and free cysteine of the RBD (Example 1.1) expressed and purified in CHO. Using the NHS ester group and maleimide group comprised in the SMPH, conjugation was progressed as the following Table 6.

**[Table 6]**

| Sample name | SARS-CoV-2 RBD | HBsAg | Linker | Reaction ratio (HBsAg: RBD) Molar ratio |
|---|---|---|---|---|
| Sample 3 | Wild-type | SEQ ID NO: 8 | SMPH | 1:0.7 |
| Sample 4 | Wild-type | SEQ ID NO: 8 | SMPH | 1:1 |
| Sample 5 | Wild-type | SEQ ID NO: 8 | SMPH | 1:2.1 |
| Sample 6 | Wild-type | SEQ ID NO: 8 | SMPH | 1:3.5 |
| Sample 7 | Wild-type | SEQ ID NO: 8 | SMPH | 1:7 |

More specifically, the SARS-CoV-2-RBD was reacted to the HBsAg VLP activated by treating SMPH (10X to 20X molar excess compared to HBsAg) according to the corresponding reaction ratio for 4 hours.

### Example 2: Confirmation of SARS-CoV-2 RBD-HBsAg VLP conjugate formation

As the result of conjugation of the SARS-CoV-2 RBD and HBsAg VLP using a chemical linker (crosslinking agent) progressed as above, formation of the conjugate in the VLP form was confirmed by DLS (Dynamic Light Scattering; zetasizer pro (Malvern)) and TEM (Transmission Electron Microscopy; JEM 1400Flash-120kV acceleration voltage).

As it is known that the SARS-CoV-2 RBD binds to ACE2-ED (extracellular domain of human Angiotensin Converting Enzyme 2 protein), in order to confirm whether the antigen protein SARS-CoV-2 RBD is displayed on the surface of the conjugate in the VLP form and binds to ACE2-ED, binding of the SARS-CoV-2 RBD-HBsAg VLP conjugate and ACE2-ED protein was confirmed by ACE2 binding assay. The ACE2 binding assay was performed using Octet^{®} Qke (Sartorius; Sensor: Protein A sensor (FORTEBIO); Ligand: ACE2-Fc (LG chem); analysis concentration: 100 ug/mL) according to the manufacturer's manual.

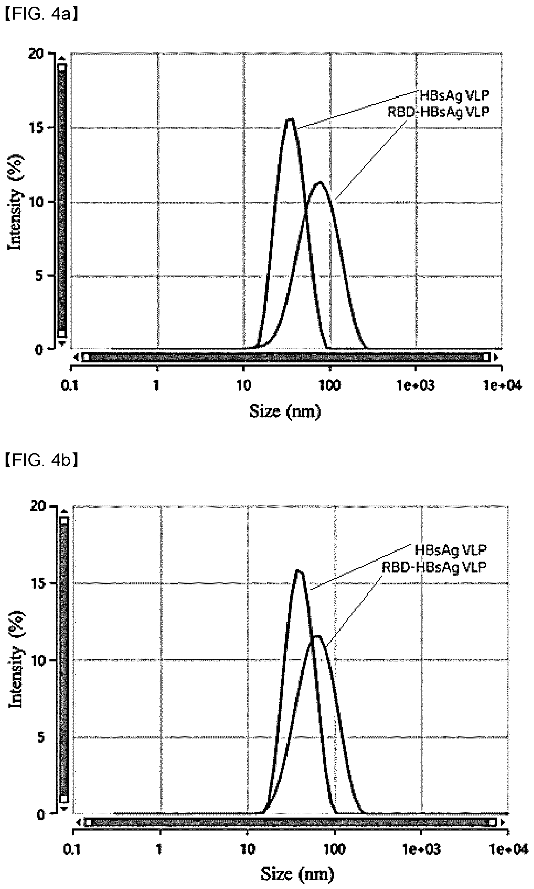

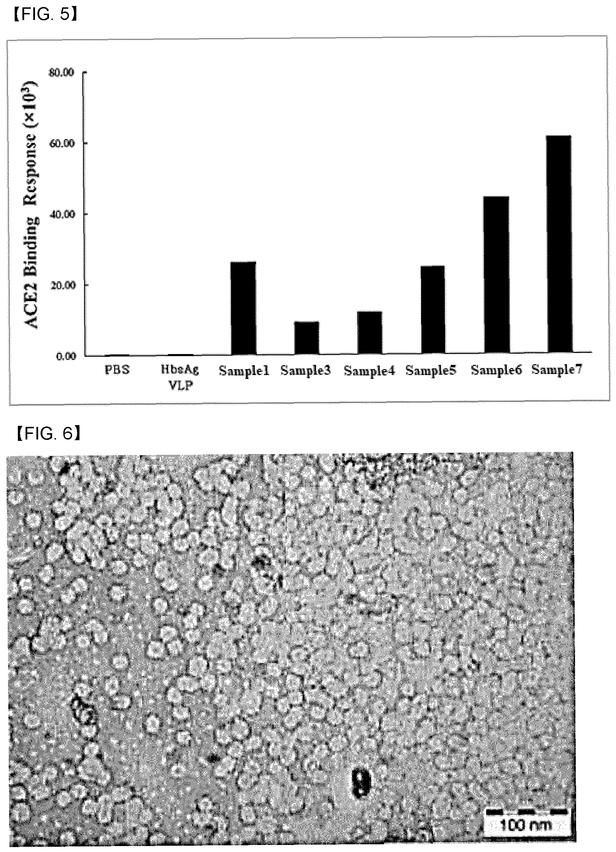

The result obtained by the analysis was shown in Table 7, and FIG. 4a (DLS: GA conjugation; Sample 1), FIG. 4b (DLS: SMPH conjugation; Sample 5), FIG. 5 (ACE2 binding assay), and FIG. 6b (TEM image of RBD-HBsAg VLP conjugate (Sample 4)):

**[Table 7]**

| Sample name | Linker | DLS | ACE2 Binding activity (100ug/uL) | HepB ELISA (R. P) |
|---|---|---|---|---|
| PBS | N.A | - | 0.26 | - |
| HBsAg VLP | N.A | 36.3 ± 2.1 | 0.24 | 1.0 |
| Sample 1 | GA | 65.7 (↑ 31.5) | 26.2 | 0.11 |
| Sample 2 | GA | 58.8 (T 24.6) | N.T. | 0.06 |
| Sample 3 | SMPH | 53.9 (T 15.5) | 8.9 | 0.011 |
| Sample 4 | SMPH | 43.5 (↑ 8.6) | 11.8 | 0.061 |
| Sample 5 | SMPH | 55.1 (T 16.7) | 24.7 | 0.011 |
| Sample 6 | SMPH | 55.7 (T 17.3) | 44 | 0.011 |
| Sample 7 | SMPH | 55.4 (T 17.0) | 60.9 | 0.011 |

| | | | | |
|---|---|---|---|---|
| (N.T.: not tested) | | | | |

As described above, it can be confirmed that the conjugate has a larger particle size than the HBsAg VLP by the DLS result (a larger number means a larger particle size), and this means that the RBD is conjugated to the HBsAg VLP surface, and the larger the difference (numerical values in parentheses) in particle size with the HBsAg VLP, the greater the number of the conjugated RBDs. In addition, the ACE2 Binding Activity result shows the amount of the RBD conjugated (exposed) to the surface (the higher the ACE2 Binding Activity numerical value, the greater the amount of the RBD exposed to the surface), and in case of HepB ELISA, the more the RBDs conjugated to the surface, the lower the level of HBsAg exposure, resulting in a relatively low value. From the result, it was confirmed that the RBD-HBsAg VLP conjugate was successfully generated and ACE2 binding activity was well maintained in both the case of conjugation using GA as a linker (crosslinking agent) and the case of conjugation using SMPH.

### Example 3: Immunogenicity 1 of SARS-CoV-2 RBD-HBsAg VLP conjugate

The immunogenicity of the SARS-CoV-2 RBD-HBsAg VLP conjugate prepared in Example 1 was confirmed by an animal experiment by making a vaccine formulation using the purified conjugate and an adjuvant (aluminum hydroxide). The vaccine formulation used in the test was prepared by the following composition:

**[Table 8]**

| Formulati on | SARS-CoV-2 RBD-HBsAg VLP conjugate | RBD-HBsAg VLP conjugate content | Adjuvant (kind/content) | Buffer |
|---|---|---|---|---|
| Formulati on 1 | Sample 1 | 200 ug/mL | Aluminum 1.0 mg/mL | Phosphate buffer (pH 6.4) |
| Formulati on 2 | Sample 3 | 200 ug/mL | Aluminum 1.0 mg/mL | Phosphate buffer (pH 6.4) |
| Formulati on 3 | Sample 4 | 200 ug/mL | Aluminum 1.0 mg/mL | Phosphate buffer (pH 6.4) |
| Formulati on 4 | Sample 5 | 200 ug/mL | Aluminum 1.0 mg/mL | Phosphate buffer (pH 6.4) |
| Formulati on 5 | Sample 6 | 200 ug/mL | Aluminum 1.0 mg/mL | Phosphate buffer (pH 6.4) |
| Formulati on 6 | Sample 7 | 200 ug/mL | Aluminum 1.0 mg/mL | Phosphate buffer (pH 6.4) |

For comparison, SARS-CoV-2 RBD (wild-type; SEQ ID NO: 2; Example 1.1) was prepared.

Antibody formation against the RBD-HBsAg VLP conjugated by the method such as ELISA and the like was confirmed by single administration or repeated administration of the formulation prepared as above of 100 µL each to an animal and blood collection. In addition, using serum collected from an animal immunized with the SARS-CoV-2 RBD-HBsAg VLP conjugate and SARS-CoV-2 or SARS-CoV-2 pseudovirus, the neutralizing antibody titer was measured by in vitro neutralization assay, and thereby, the virus prevention effect was experimented.

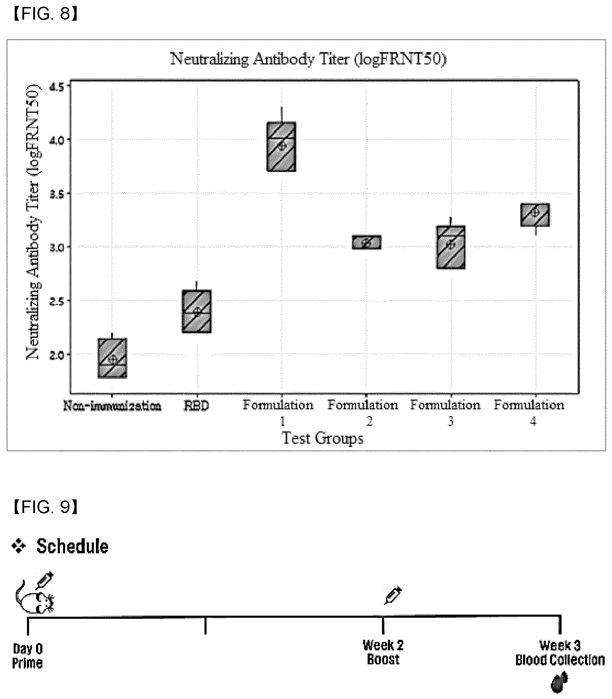

More specifically, Balb/c mice (F, 6 weeks old, 17~19g, 5 mice/group) were immunized at 0 and 2 weeks (See Table 9), and after collecting blood at 3 weeks, the binding antibody titer and neutralizing antibody titer were analyzed (See FIG. 9). The binding antibody titer was analyzed using Gyros system, and for the neutralizing antibody titer, the neutralizing ability against the wild-type SARS-CoV-2 virus (Wuhan strain, NCCP 43326) was measured by FRNT (Focus Reduction Neutralization test) method.

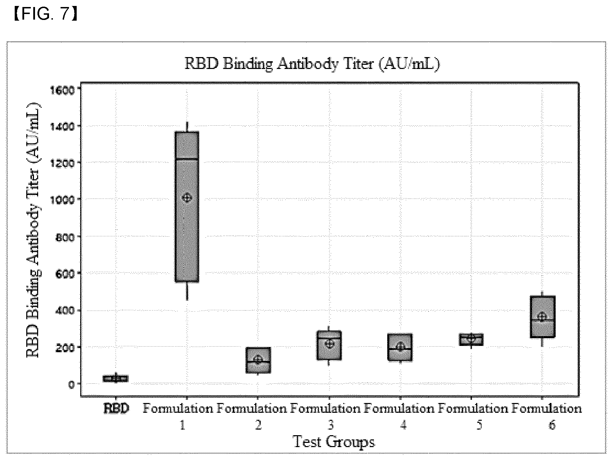

The obtained result was shown in Table 9 and FIG. 7 (RBD binding antibody titer) and FIG. 8 (neutralizing antibody titer):

**[Table 9]**

| **Test group** | **Administered protein content per mouse (ug)** | **Binding antibody titer average (AU/mL)** | **Neutralizing antibody titer (FRNT50) (average)** |
|---|---|---|---|
| | | **RBD (Geomean)** | |
| Non-immunization | - | N.D. | 208 |
| RBD | 10 | 21 | 272 |
| Formulation 1 (Sample 1) | 20 | 922 | 10240 |
| Formulation 2 (Sample 3) | 20 | 108 | 1088 |
| Formulation 3 (Sample 4) | 20 | 200 | 1152 |
| Formulation 4 (Sample 5) | 20 | 184 | 2176 |
| Formulation 5 (Sample 6) | 20 | 240 | N.T. |
| Formulation 6 (Sample 7) | 20 | 344 | N.T. |

| | | | |
|---|---|---|---|
| (protein: RBD-HBsAg VLP conjugate or RBD; N.T.: not tested, N.D.: not detected) | | | |

As can be confirmed in the above result, the SARS-CoV-2 RBD-HBsAg VLP conjugated according to the present invention showed significantly high binding antibody titer and neutralizing antibody titer compared to the SARS-CoV-2 RBD, and based on this result, it is expected that the SARS-CoV-2 RBD-HBsAg VLP conjugate has an excellent in vivo antibody production rate, and thus, it can be advantageously applied as a vaccine against coronavirus, particularly, SARS-CoV-2 (COVID-19) virus.

### Example 4: Immunogenicity 2 of SARS-CoV-2 RBD-HBsAg VLP conjugate

In order to confirm the immunogenicity by the kind of the adjuvant of the SARS-CoV-2 RBD-HBsAg VLP conjugate prepared in Example 1 (Sample A of Table 5), it was confirmed by an animal experiment by making vaccine formulations using various adjuvants. The vaccine formulations used in the animal test were prepared by the following composition:

**[Table 10]**

| Formulatio n | Description | Adjuvant | | | Antigen | |
|---|---|---|---|---|---|---|
| | | AlOH | AddaSO 3 | AddaVa x | RBD (µg/ml) | HBsAg-RBD conjugate (Sample A) (µg/ml) |
| - | Negative Control | - | - | - | - | - |
| Formulatio n 7 | RBD/AlOH | Aluminu m 1.0mg/m L | - | - | 40µg/ml | - |
| Formulatio n 8 | HBsAg-RBD/AlOH | Aluminu m 1.0mg/m L | - | - | - | 40µg/ml |
| Formulatio n 9 | HBsAg=RBD/AddaS0 3 | - | 500µl/ml | - | - | 40µg/ml |
| Formulatio n 10 | HBsAg=RBD/AddaVax | - | - | 500µl/ml | - | 40µg/ml |

| | | | | | | |
|---|---|---|---|---|---|---|
| (Buffer: PBS (3.5 mM Na₂HPO₄, 6.5 mM KH₂PO₄, 8.5 g/L NaCl and water for injection) use; Aluminum Hydroxide (AlOH) content is described based on the aluminum weight; Negative control: Non immunization) AddaS03: oil-in-water nano-emulsion adjuvant, comprising AddaS03^{™} (InvivoGen), squalene and DL-α-tocopherol at the same ratio and polysorbate 80 (Tween^{®} 80) as a surfactant; based on ASO3; AddaVax: AddaVax^{™} (InvivoGen), oil-in-water nano-emulsion adjuvant, comprising AddaVax^{™} (InvivoGen), squalene and polysorbate 80 (Tween^{®} 80) and sorbitan trioleate (Span^{®} 85) as a surfactant; based on MF59) | | | | | | |

For comparison, the SARS-CoV-2 RBD (wild-type; SEQ ID NO: 2; Example 1.1) was prepared (Formulation 7).

Formulations 7 to 10 prepared as above were administered once or repeatedly administered by 100 µL each into an animal and blood was collected, thereby confirming the antibody production against the RBD-HBsAg VLP conjugated by the method such as ELISA and the like.

More specifically, Balb/c mice (F, 6 weeks old, 10 mice/group) were immunized at 0 and 2 weeks (See Table 11), and after blood collection at 3 weeks, the neutralizing antibody titer was analyzed (See FIG. 9). For the neutralizing antibody titer, the neutralizing ability against wild-type SARS-CoV-2 virus (Wuhan strain, NCCP 43326) was measured by microneutralization assay.

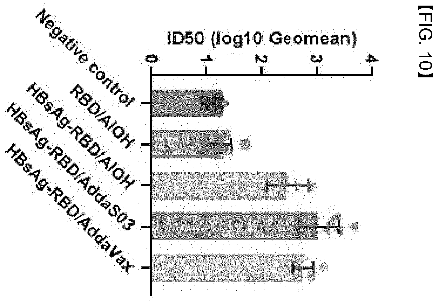

The obtained result was shown in Table 11 and FIG. 10 (average neutralizing antibody titer):

**[Table 11]**

| Formulation | Description | Adjuvant | | | Antigen | | Average neutralizin g antibody titer (ID50(Geo mean)) |
|---|---|---|---|---|---|---|---|
| | | AlOH (µg/ds) | AddaSO 3 (µl/ds) | AddaVa x (µl/ds) | RBD (µg/ds) | HBsAg-RBD conjugate (Sample A) (µg/ds) | |
| Negative Control | Negative Control | | | | | | 14.7 |
| Formulation 7 | RBD/AlOH | 100 | | | 4 | | 17.3 |
| Formulation 8 | HBsAg-RBD/AlOH | 100 | | | | 4 | 302.0 |
| Formulation 9 | HBsAg-RBD/Adda S03 | | 50 | | | 4 | 1111.1 |
| Formulation 10 | HBsAg-RBD/Adda Vax | | | 50 | | 4 | 579.9 |

As shown in Table 11 and FIG. 10, the SARS-CoV-2 RBD-HBsAg VLP conjugate according to the present invention showed the significantly high neutralizing antibody titer compared to the SARS-CoV-2 RBD, in all the cases of being used in combination with all the kinds of adjuvants tested.

From the above description, those skilled in the art to which the present invention pertains will understand that the present invention may be embodied in other specific forms without changing the technical spirit or essential characteristics thereof. In this regard, it should be understood that the examples described above are illustrative and not restrictive in all respects. The scope of the present invention should be construed as all changed or modified forms derived from the meaning and scope of the claims described below and equivalents thereof rather than the above detailed description are included in the scope of the present invention.

## Claims

1. A SARS-CoV-2 RBD-HBsAg conjugate, comprising a receptor binding domain (RBD) of a spike protein of SARS-CoV-2 (SARS-CoV-2 RBD), a hepatitis B surface antigen (HBsAg) and a bifunctional chemical crosslinking agent,
wherein the bifunctional chemical crosslinking agent is at least one selected from the group consisting of glutaraldehyde, SMPH (succinimidyl-6-((b-maleimidopropionamido)hexanoate), SMPB (Succinimidyl 4-(p-maleimidophenyl)butyrate), Sulfo-SMPB, Sulfo-EMCS [Sulfo-(N-ε-maleimidocaproyl-oxysulfosuccinimide ester)], Sulfo-GMBS [Sulfo-(N-γ-maleimidobutyryl-oxysuccinimide ester)], and SM[PEG]n (NHS-PEGn-Maliemide).

2. The SARS-CoV-2 RBD-HBsAg conjugate according to claim 1, wherein the SARS-CoV-2 RBD comprises an amino acid sequence selected from the group consisting of SEQ ID NOs: 2 to 11.

3. The SARS-CoV-2 RBD-HBsAg conjugate according to claim 1, wherein the hepatitis B surface antigen comprises an amino acid sequence selected from the group consisting of SEQ ID NOs: 12 to 14.

4. The SARS-CoV-2 RBD-HBsAg conjugate according to claim 1, wherein the bifunctional chemical crosslinking agent is glutaraldehyde or SMPH (succinimidyl-6-((b-maleimidopropionamido)hexanoate).

5. A virus-like particle (VLP), comprising the SARS-CoV-2 RBD-HBsAg conjugate of any one claim of claim 1 to claim 4.

6. A vaccine composition against coronavirus, comprising the SARS-CoV-2 RBD-HBsAg conjugate of any one claim of claim 1 to claim 4, or a virus-like particle comprising the SARS-CoV-2 RBD-HBsAg conjugate.

7. The vaccine composition according to claim 6, wherein the coronavirus is SARS-CoV-2 virus.

8. The vaccine composition according to claim 6, further comprising an adjuvant.

9. The vaccine composition according to claim 8, wherein the adjuvant is one or more kinds selected from the group consisting of an aluminum ion-containing adjuvant, a calcium ion-containing adjuvant, a biocompatible oil-based emulsion adjuvant, a liposome-containing adjuvant, a lipid-containing adjuvant, a nucleic acid-containing adjuvant, a peptide or protein-containing adjuvant, a plant extract-containing adjuvant, a saponin-derived adjuvant, a nucleic acid-containing adjuvant, a bacteria or virus-containing adjuvant, a cytokine-containing adjuvant and a biocompatible polymer-containing adjuvant.

10. A pharmaceutical composition for prevention of coronavirus infection or disease related to coronavirus infection, comprising the SARS-CoV-2 RBD-HBsAg conjugate of any one claim of claim 1 to claim 4, or a virus-like particle comprising the SARS-CoV-2 RBD-HBsAg conjugate.

11. The pharmaceutical composition for prevention according to claim 10, wherein the coronavirus is SARS-CoV-2 virus.

12. A pharmaceutical composition for treatment of coronavirus infection or disease related to coronavirus infection, comprising
an antibody binding to the SARS-CoV-2 RBD-HBsAg conjugate of any one claim of claim 1 to claim 4, or a virus-like particle comprising the SARS-CoV-2 RBD-HBsAg conjugate, or
blood or serum isolated from a subject to which the SARS-CoV-2 RBD-HBsAg conjugate, or a virus-like particle comprising the SARS-CoV-2 RBD-HBsAg conjugate is administered.

13. The pharmaceutical composition for treatment according to claim 12, wherein the antibody is isolated from blood or serum isolated from a subject to which the SARS-CoV-2 RBD-HBsAg conjugate, or a virus-like particle comprising the SARS-CoV-2 RBD-HBsAg conjugate is administered.

14. The pharmaceutical composition for treatment according to claim 12, wherein the coronavirus is SARS-CoV-2 virus.
